# EUROPEAN PATENT APPLICATION

(11) **EP 4 053 148 A1**
(43) Date of publication of application: **07.09.2022**
(21) Application number: 21159994.9
(22) Date of filing: 01.03.2021
(51) Int. Cl.: C07K 14/21, C22B 3/00, C12R 1/38

(54) **NOVEL PSEUDOMONAS STRAIN FOR METAL RECOVERY**

(71) Applicant: BRAIN Biotech AG, 64673 Zwingenberg (DE)
(72) Inventor: GABOR, Esther, 64673 Zwingenberg (DE); SCHULZE, Renate, 40489 Düsseldorf (DE)
(74) Representative: Daniels, Stefanie Lisa

(57) **Abstract**

The present invention relates to a species of genus *Pseudomonas* identified as *Pseudomonas* BR87641, termed *Candidatus Pseudomonas pretiosorbens,* having Accession Deposit Number DSM 33684.

## Description

### FIELD OF INVENTION

The invention relates to an isolated *Pseudomonas* bacteria capable of the recovery of metals, a new species of genus *Pseudomonas,* termed *Candidatus Pseudomonas pretiosorbens,* the use thereof for metal recovery, a composition containing the new species and an isolated nucleic acid.

### STATE OF THE ART

A number of living microorganisms, but also nonviable, inactivated cells have the ability to bind metal ions or nanoparticles. Metal binding can occur via adsorption to the cell surface or via active intracellular accumulation of metals that is often referred to as biosorption or bioadsorption. In the case of nonviable, inactivated cells - metal ion or nanoparticle binding is believed to occur exclusively via surface adsorption. The biosorption capacity as a general characteristic of biomass results from the presence of chelating groups (e.g. carboxyl-, amide-hydroxyl-, phosphate-, and thiol-groups) contributed by carbohydrates, lipids and proteins that are displayed on the cell surface. It has been described that amounts of metals of up to 50 % of the cell dry weight can be accumulated by biomass (Vieira and Volesky, 2000). United States Patent 1991/5055402 describes a process for removing metal ions from aqueous solution, using a matrix prepared from metal-binding microorganisms that have been heat-inactivated at temperatures of 300-500°C. However, specific binding mechanisms by organic surface structures are obviated by this procedure.

EP 0673350 B1 describes the accumulation of metals, including some rare earth elements such as lanthanium and yttrium, by reacting phosphate ions generated by a microorganism and metals to polyphosphates. Accumulation of the metal-polyphosphates by the microorganism of the genus *Acetobacter* makes the metals accessible to precipitation and depletion thus enabling purification of metal-polluted water. WO 1991/003424 describes a biomining procedure for leaching of gallium and germanium from ores using an admixture of bacteria, culture medium and crushed ore. However, no process has been described to date that could be used to recover platinum group metals in significant amounts from liquid waste materials using a microorganism from which biomass can be produced in a large scale.

Karavaiko et al., Biotechnology Letters, 18 (1996) 1291-1296 describes biosorption of scandium and yttrium from solutions using biomass of *Saccharomyces cerevisiae* and *Aspergillus terreus.* MINT Technologies (WO 2018/080326 A1 and WO 2018/084723 A1) describe methods for recovering metals, especially gold, from solid feedstocks using *Cupriavidus metallidurans, Bacillus subtilis* and *Pseudomonas putida* as microorganisms. Yet, there is a need for microorganisms which are capable of preferential binding and separation of metals, particularly precious metals, specifically platinum group metals. Furthermore, there is a need for non-pathogenic robust microorganisms from which biomass can be produced in a large scale.

### DESCRIPTION OF THE INVENTION

This object is solved by claim 1 of the present invention, which is directed to an isolated *Pseudomonas* bacteria capable of the recovery of metals, having at least 94% identity to SEQ ID No 2.

Surprisingly the inventors have found that the isolated *Pseudomonas* bacteria according to the invention has a lot of advantageous characteristics, for example
(a) being able to recover precious metals and/or platinum group metals from diverse liquid material streams such as mine drainages, communal or industrial waste waters, process streams, for example from metal refining or recycling process as well as supernatants from bioleaching processes
(b) having surface structures that result in recovery and concentration of precious metals and/or platinum group metals at a very broad pH range, ranging from acid leachates with pH <1, over neutral solutions to alkaline leachates with pH >13
(c) recovering precious metals and/or platinum group metals with living cells, inactivated cells and living and/or inactivated cells that are immobilized
(d) having a high tolerance towards external conditions, such as toxic elements and compounds, heat and pH
(e) being a risk group 1 organism that can be cultivated at high cell density

In a preferred embodiment according to the invention, the isolated *Pseudomonas* bacteria has at least 96% identity to SEQ ID No 2. In a more preferred embodiment according to the invention, the isolated *Pseudomonas* bacteria has at least 98% identity to SEQ ID No 2.

In a most preferred embodiment according to the invention, the isolated *Pseudomonas* bacteria is a species of genus *Pseudomonas,* identified as *Pseudomonas* sp. BR8764 termed *Candidatus Pseudomonas pretiosorbens,* having Accession Deposit Number DSM 33684.

As used herein the terms "species", "strain" (with regard to *Pseudomonas* sp. BR8764) and *"Pseudomonas pretiosorbens"* can be used synonymously since the terms all relate to *Candidatus Pseudomonas pretiosorbens,* identified as *Pseudomonas* sp. BR8764 (having Accession Deposit Number DSM 33684).

Platinum group metals according to the invention are defined as Ruthenium (Ru), Rhodium (Rh), Palladium (Pd)) (light platinum metals) and Osmium (Os), Iridium (Ir), Platinum (Pt) (heavy platinum metals) and their mixtures.

Precious metals according to the invention are defined as being selected from the platinum group metals and additionally gold and silver and their mixtures.

As used herein, the singular form "a," "an," and "the" include plural references unless the context clearly dictates otherwise. For example, the term "a compound" or "at least one compound" may include a plurality of compounds, including mixtures thereof. Thus, for example, reference to "liquid material," "the liquid material," or "a liquid material" also includes a plurality of liquid materials; use of the term "a nucleic acid" optionally includes, as a practical matter, many copies of that nucleic acid molecule.

As used herein, the term "about" indicates that a value includes the inherent variation of error for the method being employed to determine a value, or the variation that exists among.

As used herein the term "biosorption" or "biosorption-based processes" means the recovery and concentration of metals from materials. In a preferred embodiment according to the invention, the recovery from platinum group metals and/or precious metals from materials. In a further preferred embodiment, the recovery from platinum group metals and/or precious metals from liquid materials.

### FIGURES

**FIG. 1****:** is a distance matrix which was calculated by the Jukes-Cantor correction method using filter pju6q4wa (*E. coli* position 100 to 1427 using arb-program, Ludwig et al., 2004) based on partial 16S rRNA gene sequences. Tree Dendrogram was generated by neighbour joining method.
**FIG. 2****:** shows sequence similarity (expressed as percentage) of partial gene sequences (912 nt) of housekeeping gene *rpo*B (program arb, filter SAI_AJ7174 912 nt, Ludwig et al, 2004).
**FIG. 3****:** shows sequence similarity (expressed as percentage) of partial gene sequences (560 nt) of housekeeping gene *rpo*D (program arb, filter cFM25190 560 nt Ludwig et al, 2004).
**FIG. 4****:** shows the average nucleotide identity based on BLAST algorithm (ANIb). ANIb analysis (percentage identity) was performed using program PYANI (Pritchard et al. (2016)).
**FIG. 5****:** shows analyzation of physiological traits of *Pseudomonas* sp. BR8764 with the standardized system for identification of non-fastidious, non-enteric Gram-negative rods that is API20 NE (BIOMÈRIEUX) and carbon utilization tests based on BIOLOG PM1 and PM2A plates (BIOLOG) according to the manufacturer's instructions. Results were compared with selected results of relevant strains.
**FIG. 6****:** shows the 16S rRNA gene sequence of *Pseudomonas* sp. BR8764, which is SEQ ID No. 1.

**BIOSORPTION:** As already stated above, the isolated *Pseudomonas* bacteria according to the invention is capable of recovering metals, particularly precious metals, more specifically platinum group metals from liquid materials by biosorption.

The term "liquid material" means solutions of metal ions, metal complexes or metal nanoparticles that are based on water, non-aqueous solvents or organic solvents or a mixture thereof. Water-based liquid materials are for example mine drainages, communal or industrial waste waters, natural waterbodies and aquifers, as well as acidic and alkaline bioleaching solutions. Liquid materials based on concentrated or diluted non-aqueous or organic solvents or mixtures thereof usually originate from chemical processes as employed in the mining, metal refining and metal recycling industries. In a preferred embodiment according to the invention, the liquid material is water-based.

***Pseudomonas* sp. BR8764:** According to a preferred embodiment of the invention, *Pseudomonas* sp. BR8764 is provided as new organism. This organism can be used for recovering metals, particularly precious metals, more specifically platinum group metals from liquid materials. Surprisingly, this organism is capable of biosorption of target metals also from materials that contain elevated levels of toxic metals such as lead, chromium and cadmium. This allows the conclusion that the biomass of organism is not only able of biosorption of metals or metal recovery, particularly precious metals, more specifically platinum group metals from liquid materials but the biomass has also a high tolerance against external conditions such as toxic elements and compounds, heat and extreme pH conditions.

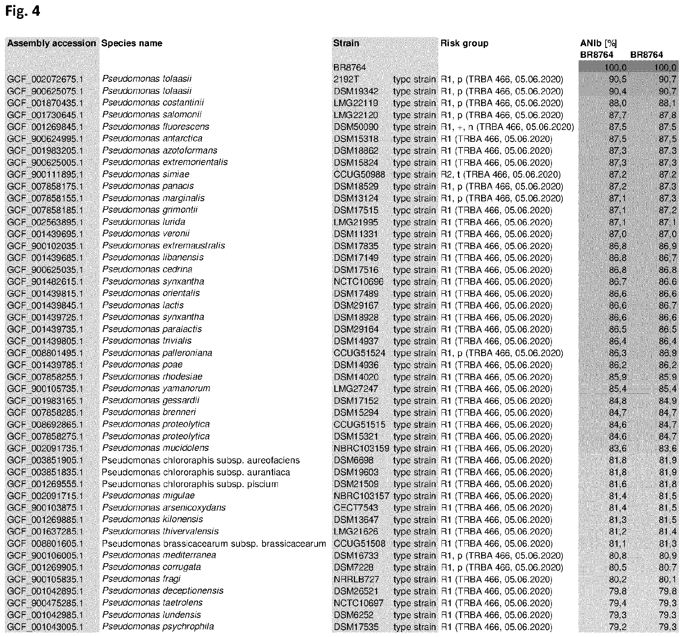

It has been found that *Pseudomonas* sp. BR8764 represents a novel species of the genus *Pseudomonas.* Classification of *Pseudomonas* sp. BR8764 to a novel species of genus *Pseudomonas* was supported by phenotypic (API, BIOLOG) and genotypic data. Genomic data were used for further phylogenetic analysis of partial sequences of the housekeeping genes *rpoB, rpoD* and *gyrB* and calculation of average nucleotide identity (ANI) based on BLAST algorithm using scaffold genomes. As shown in Fig. 4 ANIb similarity values are below 91%. These results further underline that *Pseudomonas* sp. BR8764 represents a novel species of the genus *Pseudomonas. Pseudomonas* strains sharing at least 94% ANI to scaffold genome of *Pseudomonas* sp. BR8764 are regarded to be part of the new group of *Pseudomonas* (Goris et al., 2007).

Furthermore, the physiology of *Pseudomonas* sp. BR8764 was found to be distinct from *Pseudomonas* species known so far as indicated by the API (Biomerieux API 20 NE) and BIOLOG (BIOLOG PM1, PM2A) assays.

**DEPOSIT OF BIOLOGICAL MATERIAL:** The isolated and identified species *Pseudomonas* sp. BR8764 was deposited on November 5th, 2020, under the provisions of the Budapest Treaty in the Leibniz Institute DSMZ - German Collection of Microorganisms and Cell Cultures. It has been assigned to the Accession number DSM 33684.

In a preferred embodiment according to the invention, the isolated *Pseudomonas* bacteria according to the invention, preferably the new species *Pseudomonas* sp. BR8764, belongs to risk group 1. The term "risk group" is a classification system which is used in many countries for the classification of microorganisms. The affiliation to a risk group depends for example on the following factors
- Pathogenicity of the organism
- Mode of transmission and host range
- Availability of effective preventive measures (e.g. vaccines)
- Availability of effective treatment (e.g. antibiotics)
All microorganisms which belong to risk group 1 are not associated with disease in healthy adult humans (see NIH Guidelines in Recombinant DNA, April 2002).

In an embodiment according to the invention the ribosomal DNA of *Pseudomonas* sp. BR8764 has SEQ ID No. 1.

**USE:** In one embodiment according to the invention, the isolated *Pseudomonas* bacteria having at least 94% identity to SEQ ID No. 2 is used for the recovery of metals, particularly precious metals, specifically platinum group metals from liquid material streams.

Preferably, the liquid material streams are selected from mine drainage waters, communal or industrial waste waters, process streams, for example from metal refining or recycling processes as well as supernatants from bioleaching processes.

As already stated above, the platinum group metals are are defined as Ruthenium (Ru), Rhodium (Rh), Palladium (Pd)) (light platinum metals) and Osmium (Os), Iridium (Ir), Platinum (Pt) (heavy platinum metals) and their mixtures.

In a preferred embodiment according to the invention, the isolated *Pseudomonas* bacteria has at least 96% identity to SEQ ID No 2. In a more preferred embodiment according to the invention, the isolated *Pseudomonas* bacteria has at least 98% identity to SEQ ID No 2.

Furthermore preferred is the use of *Pseudomonas* sp. BR8764 for the recovery of metals, particularly precious metals, specifically platinum group metals from liquid material streams.

In one preferred embodiment, *the Pseudomonas* bacteria according to the invention can be used in the form of free biomass. The biomass can consist of living cells or inactivated cells or cross-linked cells. In another preferred embodiment, *the Pseudomonas* bacteria can be used as a composition that contains the cells within or on the surface of a sold carrier material or cross-linked cells as described as follows.

**COMPOSITION:** A further embodiment according to the invention relates to a composition comprising:
(a) the isolated *Pseudomonas* bacteria having at least 94% identity to SEQ ID No 2 in amounts effective to facilitate metal recovery, particularly metal recovery of precious metals and/or platinum group metals;
(b) at least one liquid material.

In a preferred embodiment, at least one liquid material (b) is selected from secondary resources, wherein the secondary resource is selected from waste waters from mine drainages, communal or industrial waste waters, process streams, for example from metal refining or recycling processes as well as supernatants from bioleaching processes.

As already stated above, the platinum group metals are are defined as Ruthenium (Ru), Rhodium (Rh), Palladium (Pd)) (light platinum metals) and Osmium (Os), Iridium (Ir), Platinum (Pt) (heavy platinum metals) and their mixtures.

In a preferred embodiment according to the invention, the *Pseudomonas* bacteria according to the invention is heat inactivated at temperatures >70°C. In another embodiment the polymer used in b) is for example an alginate, a poly vinyl alcohol-alginate, gelrite, a silicate or a sol gel matrix. In a preferred embodiment according to the invention, the isolated *Pseudomonas* bacteria has at least 96% identity to SEQ ID No 2. In a more preferred embodiment according to the invention, the isolated *Pseudomonas* bacteria has at least 98% identity to SEQ ID No 2. Preferably, the *Pseudomonas* bacteria is selected from *Pseudomonas* sp. BR8764.

Further preferred is a composition comprising
(a) a preparation of living or inactivated cells of the *Pseudomonas* bacteria having at least 94% identity to SEQ ID No 2 in amounts effective to allow the recovery of metals, particularly platinum group metals or others, wherein the preparation of the *Pseudomonas* bacteria is embedded in an organic or inorganic polymer or a solid material, wherein the organic or inorganic polymer or the solid material serve as a carrier for the preparation of the *Pseudomonas* bacteria; and
(b) at least one liquid material.

Suitable materials for solid carriers are ceramic materials such as bentonite or materials such as polyurethane foam and plaster of paris, gypsum or nylon.

In a further embodiment of the invention, the preparation of living or inactivated cells of the *Pseudomonas* bacteria is cross-linked with, for example, glutaraldehyde or polyethyleneimine, or oxalic acid and polyethyleneimine.

In a preferred embodiment according to the invention, the isolated *Pseudomonas* bacteria has at least 96% identity to SEQ ID No 2. In a more preferred embodiment according to the invention, the isolated Pseudomonas bacteria has at least 98% identity to SEQ ID No 2. Preferably, the *Pseudomonas* bacteria is selected from *Pseudomonas* sp. BR8764.

**METHOD:** One embodiment according to the invention relates to a method for recovering metals from liquid material with the *Pseudomonas* bacteria having at least 94% identity to SEQ ID No 2. In a preferred embodiment according to the invention, this method comprises or consists of the following steps:
(a) incubation of biomass of the *Pseudomonas* bacteria with the liquid material;
(b) separation of biomass and metal-depleted liquid material; (c) recovery of metals from biomass; in this step, metals are released from the biomass by e.g. elution or incineration of the combustible biomass.

In step (a), the metals ions, complexes or nanoparticles bind to the biomass. In step (b), the metal ions, complexes or nanoparticles are concentrated and separated from the liquid material. This step can for example be realized by standard solid-liquid separation methods, for example by centrifugation or sedimentation.

In a preferred embodiment according to the invention, the isolated *Pseudomonas* bacteria has at least 96% identity to SEQ ID No 2. In a more preferred embodiment according to the invention, the isolated *Pseudomonas* bacteria has at least 98% identity to SEQ ID No 2. Preferably, the *Pseudomonas* bacteria is selected from *Pseudomonas* sp. BR8764.

**ISOLATED NUCLEIC ACID:** A further embodiment according to the invention relates to an isolated nucleic acid having at least 94% identity to SEQ ID No. 2.

In a preferred embodiment according to the invention the isolated nucleic acid having at least 95%, at least 96%, at least 97%, at least 98%, at least 99% identity to SEQ ID No. 2. In another preferred embodiment according to the invention the isolated nucleic acid having 100% identity to SEQ ID No. 2. In other words, the isolated nucleic acid is SEQ ID No. 2.

As used herein, the term "identity" when used in relation to nucleic acids, describes the degree of similarity between two or more nucleotide sequences. The percentage of "sequence identity" between two sequences can be determined by comparing two optimally aligned sequences over a comparison window, such that the portion of the sequence in the comparison window may comprise additions or deletions (gaps) as compared to the reference sequence (which does not comprise additions or deletions) for optimal alignment of the two sequences. The percentage is calculated by determining the number of positions at which the identical nucleic acid base or amino acid residue occurs in both sequences to yield the number of matched positions, dividing the number of matched positions by the total number of positions in the window of comparison, and multiplying the result by 100 to yield the percentage of sequence identity. A sequence that is identical at every position in comparison to a reference sequence is said to be identical to the reference sequence and vice-versa. An alignment of two or more sequences may be performed using any suitable computer program. For example, a widely used and accepted computer program for performing sequence alignments is CLUSTALW vl .6 (Thompson, et al. (1994) Nucl. Acids Res., 22: 4673-4680).

SEQ ID No. 2 represents an artificial sequence. This artificial sequence was nanopore sequenced and represents the genome sequence of *Pseudomonas* sp. BR8764. Furthermore, the nanopore sequence was further polished with the Illumina technique. Nanopore sequencing is a third generation approach used in the sequencing of biopolymers- specifically, polynucleotides in the form of DNA or RNA. By using nanopore sequencing, a single molecule of DNA or RNA can be sequenced without the need for PCR amplification or chemical labeling of the sample. The DNA/RNA is transported through a pore which has the size of a nanometer. When it passes through, the voltage on this pore is changed. The change in the voltage is specific for each of the four nucleobases, whereby the sequence can be recognized. The nanopore itself consists of a recombinant protein that is embedded in a polymer membrane.

### EXAMPLES

### EXAMPLE 1: Isolation of Pseudomonas sp. BR8764

The environmental sample for isolation of *Pseudomonas* sp. BR8764 was taken in Germany in Grube Segen Gottes in Haslach on 28.02.2012. Therefore, there are no Nagoya Protocol restrictions for this strain. This strain was isolated according to the following procedure: A small amount of environmental sample was suspended in about 500 µl 0.9% NaCl solution and plated on TSB agar plates. Incubation temperature was 10°C. Appearing microbial colonies were transferred to LB agar plates and purified by a "clean streak" on fresh LB agar plates. Small scale cultivation was done at 28°C in LB medium (such as Bacto Tryptone #211705 (BD) 1% (w/v), Bacto Yeast Extract #212750 (BD) 0.5% (w/v), NaCl #141659.1221 (Applichem) 0.5% (w/v).

### EXAMPLE 2: Phylogenetic Analysis of Pseudomonas sp. BR8764 and definition of a new Candidatus of genus Pseudomonas

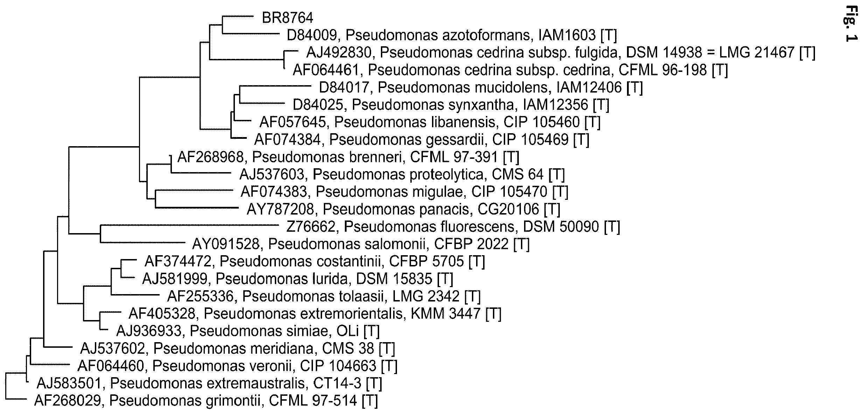

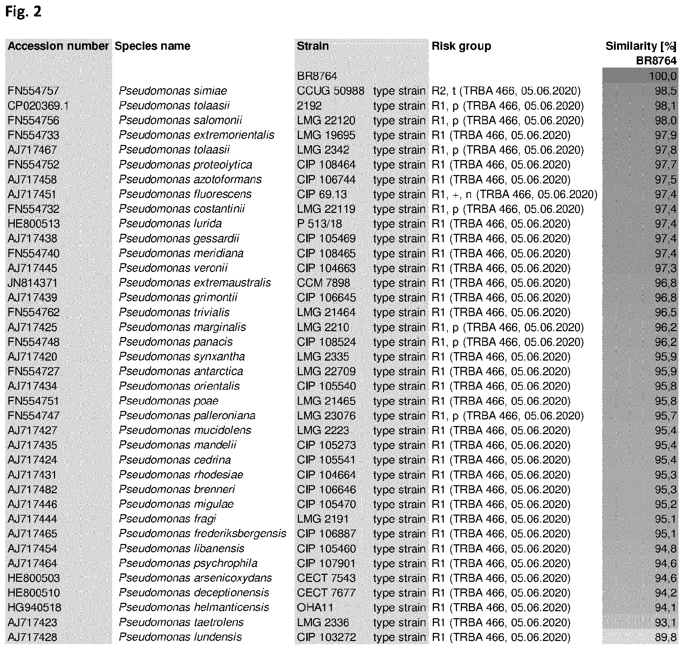

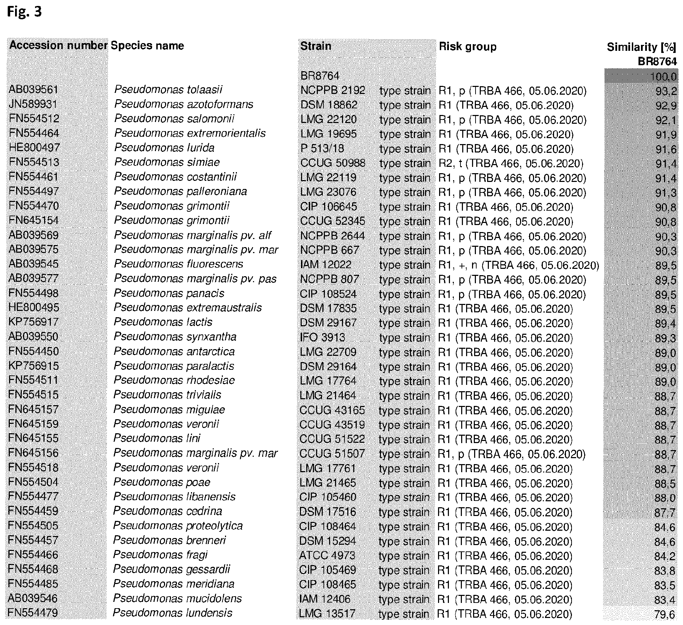

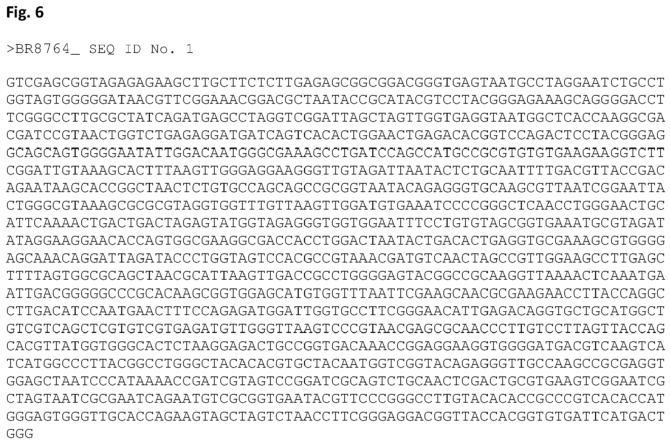

First of all the affiliation of strain *Pseudomonas* sp. BR8764 to the genus *Pseudomonas* was shown by performing 16S rRNA gene analysis of partial 16S rRNA gene sequences (Fig. 1) using program arb (Ludwig et al., 2004). The 16S rDNA sequence of *Pseudomonas* sp. BR8764 is shown in Fig. 6 and SEQ ID No. 1. To further investigate phylogenetic relationships of *Pseudomonas* sp. BR8764, housekeeping genes *rpoB* and *rpoD* were analyzed. The primers LAPS and LAPS27 were used to amplify partial *rpoB* gene (Tayeb et al., 2005). For amplification of partial sequences of *rpoD* the PsEG30F/PsEG790R primers were used (Mulet et al., 2009). As is shown in Fig. 2 maximal similarity values are in case of *rpoB* around 98%. To further investigate relationship to closest related type strains similarity of partial *rpoD* sequence was analyzed. As is shown in Fig. 3 maximal similarity values to closest related type strains are around or below 93% including all type strain sequences whose partial rpoB-Sequence shows similarity values around 97% or 98%. Low similarity values of all *rpoD* sequences indicate that *Pseudomonas* sp. BR8764 could be a new species. To further investigate phylogenetic relationship the average nucleotide identity (ANI) analysis (e.g. Richter and Rosselló-Móra, 2009), a method to substitute DNA-DNA hybridization experiments, were chosen. The species level boundary proposed was set at about 94% ANI (e.g. Richter and Rosselló-Móra, 2009). As shown in Fig. 4 ANIb similarity values are below 91%. These results further underline that *Pseudomonas* sp. BR8764 represents a novel species of the genus *Pseudomonas. Pseudomonas* strains sharing at least 94% ANI to scaffold genome of *Pseudomonas* sp. BR8764 are regarded to be part of the new group of *Pseudomonas* (Goris et al., 2007).

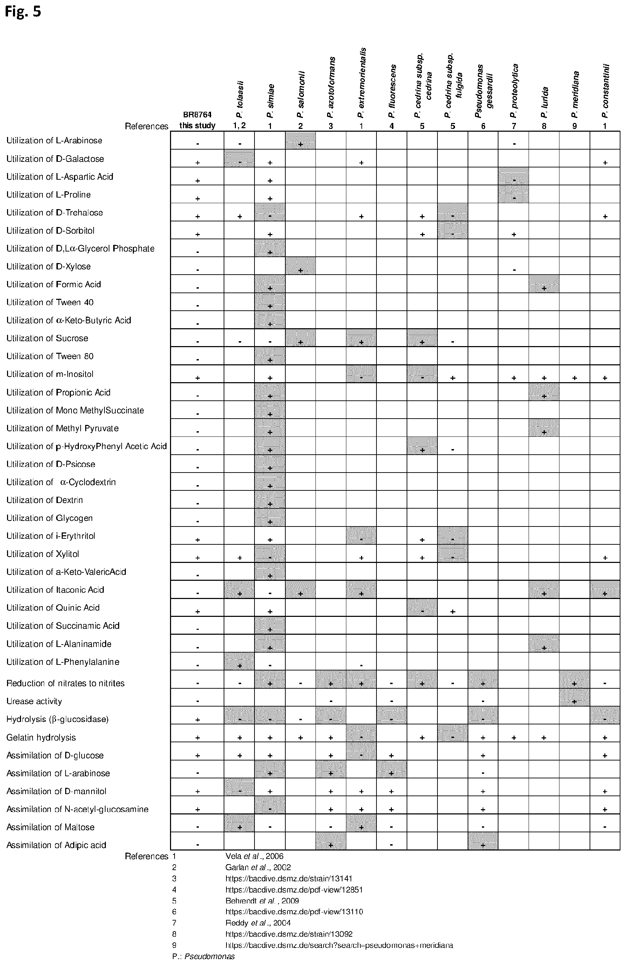

To analyze physiological traits of *Pseudomonas* sp. BR8764 the standardized system for identification of non-fastidious, non-enteric Gram-negative rods that is API 20 NE (BIOMÈRIEUX) and carbon utilization tests based on BIOLOG PM1 and PM2A plates (BIOLOG) were used according to the manufacturer's instructions. Results were compared with selected results from literature of physiological tests of relevant strains. Relevant strains were chosen based on above phylogenetic sequence analysis results (Fig. 1 to Fig. 4). Analysis revealed that the physiology of *Pseudomonas* sp. BR8764 is distinct from so far known *Pseudomonas* species as indicated by the API and BIOLOG assays (Fig. 5). *Pseudomonas* sp. BR8764 grows at 20°C, 28°C, 30°C, but shows no growth at 37°C.

These data further underline that *Pseudomonas* sp. BR8764 can be classified as a novel species of the genus *Pseudomonas* termed *Candidatus Pseudomonas pretiosorbens.*

The isolated and identified strain has been submitted on November 5th, 2020 to the Leibniz Institute DSMZ - German Collection of Microorganisms and Cell Cultures. It has the accession number DSM 33684.

### EXAMPLE 3: Recovery and concentration of precious metals using Pseudomonas sp. BR8764

First of all, *Pseudomonas* sp. BR8764 was cultured on LB agar plate for two days at 28°C. Then cells from LB agar plate were suspended in 500 µl LB-medium. 50 µl were transferred to 10 mL of Riesenberg medium (see below) with 5 g glycerol per l. After over night incubation in a shaker, 417 µl of preculture were used for inoculation of 50 ml of the same medium to a final OD of 0.05. The optical density of the culture was 3.2 after 7.67 hours. For generation of working cell bank cryo-culture, 900 µl of suspended culture were filled in a tube, mixed with 900 µl of 30% glycerol solution and frozen at -80°C. One preculture of 50 mL Riesenberg medium in 500 mL Erlenmeyer flask was inoculated with one working cell culture. After over night incubation in a shaker bioreactor culture was inoculated using preculture to get start a OD of OD 0.1. *Pseudomonas* sp. BR8764 was cultured in 2 L bioreactor (Sartorius, Biostat B) filled with about 1.8 L medium for 16 hours in batch fermentation. Riesenberg medium was prepared with 26 mM KH₂PO₄ (AppliChem, #A3620), 30 mM (NH₄)₂HPO₄ (AppliChem (#A2291), 9 mM C₆H₈O₇*H₂O (AppliChem (#A4212). Then, after autoclaving 5 mM MgSO₄*7H₂O (AppliChem, #A4101), 1 ml trace element solution (50 mM FeSO₄ *7H₂O, 10 mM MnSO₄*1H₂O, 10 mM ZnSO₄*7H₂O, 2 mM CoSO₄*7H₂O, 2 mM CuSO₄*5H₂O, 2 mM NiSO₄*6H₂O, 2 mM Na₂MoO₄*2H₂O, 2 mM Na₂SeO₃, 2 mM H₃BO₃) and 15 g glycerol per liter (Sigma-Aldrich, W252506-25KG-K) was added. pH was adjusted to 6.8 using 5M NaOH-solution. After harvesting of biomass and washing with 0.9% NaCI-solution the pellet was frozen at -80°C and lyophilized, referred to as dry matter (DM).

To generate test solutions commercially acquired ICP-standard solutions from ESI-Elemental Scientific (www.icpms.de) were diluted to about 100 ppm in either 10 [%] HCl for test solution 1 or 7.5 [%] HNO₃ for test solution 2. Following standards were used: PdCl₂ (10% v/v HCl, 1000 ppm), CGPD1-125ML; Pt (10% v/v HCl, 1000 ppm), CGPT1-125ML; RhCl₃ (15% v/v HCl, 1000 ppm) CGRH1-125ML; IrCl₃ (10% v/v HCl, 1000 ppm) CGIR1-125ML; Al (3% v/v HNO3, 1000 ppm) CGAL1-125ML; Fe (2% v/v HNO₃, 1000 ppm) CGFE1-125ML. To analyse elementary composition in test solutions ion-coupled plasma mass spectrometry (ICP-MS) was applied. Elementary composition of test solution 1 and 2 was as shown in Table 1.

**Table 1**

| Elementary composition of Test solution 1 and 2 | | | | | | |
|---|---|---|---|---|---|---|
| | **[ppm]** | | | | | |
| | **Pd** | **Pt** | **Rh** | **Ir** | **Al** | **Fe** |
| **test solution 1 (ts1)** | 100 | 102 | 98 | 97 | 105 | 103 |
| **test solution 2 (ts2)** | 98 | 99 | 97 | 91 | 108 | 100 |

Experimental set-up for biosorption experiments was as described as follows. Dry matter (DM) of biomass prepared as described above was weight into 2 mL-test tubes. Then 1.5 mL test solution were added and incubated at room temperature for 3 hours by mixing using an overhead shaker. Afterwards the samples were centrifuged for 10 min at 13300 rpm. Supernatant was taken to analyze elementary composition by ICP-MS. Metal binding to biomass was calculated by subtracting ICP-MS-element value of supernatant from value in test solution. Recovery was expressed as [%] bound as shown in Table 2.

**Table 2**

| Biosorption of precious metal on dry matter of *Pseudomonas* sp. BR8764 | | | | | | | | | | | | | |
|---|---|---|---|---|---|---|---|---|---|---|---|---|---|
| | | **[%] bound** | | | | | | | | | | | |
| | | **Pd** | | **Pt** | | **Rh** | | **Ir** | | **Al** | | **Fe** | |
| | | **ts 1** | **ts 2** | **ts 1** | **ts 2** | **ts 1** | **ts 2** | **ts 1** | **ts 2** | **ts 1** | **ts 2** | **ts 1** | **ts 2** |
| **[mg] DM** | **30** | 92 | 94 | 24 | 21 | 9 | 16 | 16 | 14 | 5 | 15 | 71 | 88 |
| | **60** | 98 | 97 | 40 | 43 | 17 | 32 | 25 | 26 | 11 | 26 | 75 | 85 |
| | **90** | 98 | 98 | 61 | 61 | 22 | 40 | 29 | 29 | 17 | 35 | 69 | 82 |
| | **120** | 97 | 96 | 79 | 82 | 26 | 45 | 34 | 36 | 20 | 34 | 59 | 73 |
| | | ts: 1.5 mL test solution | | | | | | | | | | | |

As shown in Table 2 using dry matter of *Pseudomonas* sp. BR8764 recovery of palladium above 90% was shown in acid HCl- or HNO₃-leachates. Recovery of platinum was observed up to around 80%. In HCI-acid leachate recovery of rhodium or iridium up to around 30% was shown. In HNO₃-acid leachate recovery or rhodium or iridium was even higher. Recovery of up to around 40% was determined. However, some aluminium or iron was also recovered, but preferential biosorption of Pd was observed.

## Claims

1. An isolated *Pseudomonas* bacteria capable of the recovery of metals, having at least 94% identity to SEQ ID No 2.

2. The isolated *Pseudomonas* bacteria according to claim 1, wherein the isolated *Pseudomonas* bacteria has at least 96% identity to SEQ ID No 2.

3. The isolated *Pseudomonas* bacteria according to claim 1, wherein the isolated *Pseudomonas* bacteria has at least 98% identity to SEQ ID No 2.

4. The isolated *Pseudomonas* bacteria according to claim 1, wherein the isolated *Pseudomonas* bacteria is a species of genus *Pseudomonas,* identified as *Pseudomonas* sp. BR8764 termed *Candidatus Pseudomonas pretiosorbens,* having Accession Deposit Number DSM 33684.

5. The isolated *Pseudomonas* bacteria according to at least one of the preceding claims, wherein said *Pseudomonas* bacteria is capable of the recovery of platinum group metals, metals and/or precious metal from liquid materials.

6. Use of the isolated *Pseudomonas* bacteria according to claim 1 for metal recovery.

7. Use of the isolated *Pseudomonas* bacteria according to claim 1 for metal recovery of precious metals and/or platinum group metals.

8. Use of the isolated *Pseudomonas* bacteria according to claim 1 for metal recovery from liquid material streams.

9. Use of the isolated *Pseudomonas* bacteria according to claim 8, wherein the liquid material streams are selected from mine drainage waters, communal or industrial waste waters, process streams, for example from metal refining or recycling processes as well as supernatants from bioleaching processes.

10. Use according to at least one of claims 6 to 9, wherein the platinum group metals are selected from the group consisting of palladium, platinum, ruthenium, rhodium, osmium, iridium, and their mixtures.

11. Use according to at least one of claims 6 to 10, wherein the isolated *Pseudomonas* bacteria is a species of genus *Pseudomonas,* identified as *Pseudomonas* sp. BR8764 termed *Candidatus Pseudomonas pretiosorbens,* having Accession Deposit Number DSM 33684.

12. A composition comprising:
(a) the isolated *Pseudomonas* bacteria according to claim 1 in amounts effective to facilitate metal recovery, particularly metal recovery of precious metals and/or platinum group metals;
(b) at least one liquid material.

13. The composition according to claim 12, wherein at least one liquid material (b) is selected from secondary resources, wherein the secondary resource is selected from waste waters from mine drainages, communal or industrial waste waters, process streams, for example from metal refining or recycling processes as well as supernatants from bioleaching processes.

14. The composition according to claim 12 or 13, wherein platinum group metals are selected from the group consisting of ruthenium, rhodium, palladium, osmium, iridium, platinum and their mixtures.

15. An isolated nucleic acid having at least 94% identity to SEQ ID No 2.
